# EUROPEAN PATENT APPLICATION

(11) **EP 4 706 609 A1**
(43) Date of publication of application: **11.03.2026**
(21) Application number: 25199920.7
(22) Date of filing: 03.09.2025
(51) Int. Cl.: A61F 9/02, G02C 7/02

(54) **GOGGLE LENS WITH COMPOUND CURVATURE**

(30) Priority: 04.09.2024 TW 113133524
(71) Applicant: CHIU YU, Hui-Hsuan, Tainan City 708013 (TW)
(72) Inventor: CHIU YU, Hui-Hsuan, Tainan City 708013 (TW)
(74) Representative: Stöckeler, Ferdinand

(57) **Abstract**

A goggle lens is coupled with a goggle frame (90) for enclosing a wearer's head, and includes a first lens portion (1), at least one second lens portion (2) integrally connected with a lower edge of the first lens portion (1), and a third lens portion (3) integrally formed with and extends upwardly from an upper edge of the first lens portion (1). A first convex surface (12) of the first lens portion (1) has a first vertical radius of curvature (ROC11) which is different from a second vertical radius of curvature (ROC12) of a second convex surface (22) of the second lens portion (2), and is different from a third vertical radius of curvature (ROC13) of a third convex surface (32) of the third lens portion (3) so as to provide a wearer with good visual effects.

## Description

The disclosure relates to a goggle lens, and more particularly to a goggle lens with compound curvature.

A conventional goggle disclosed in US11726351 includes a goggle frame defining an opening, and a lens assembly coupled with the goggle frame in a magnetically attractive manner. The lens assembly includes a lens covering the opening of the goggle frame. The lens has an upper portion and a lower portion. The lower portion has a varying radius of curvature along the meridian line to provide a wearer with a greater field of view when the wearer is looking in a downward direction.

However, such a goggle when used during athletic play or snow sports requiring a wide field of view, might have image inconsistency or distortion when the wearer is looking in a variety of direction (such as seeing upwardly or downwardly) through the lens. The wearer may thus be subjected to visual discomfort and dizziness, which may increase the risk of danger during athletic activities.

Therefore, an object of the disclosure is to provide a goggle lens that can alleviate at least one of the drawbacks of the prior art.

According to the disclosure, the goggle lens is adapted to be coupled with a goggle frame for enclosing a wearer's head. The goggle lens includes a first lens portion, at least one second lens portion and a third lens portion. The first lens portion includes a first convex surface that is distal to the wearer's face. The first convex surface has a first vertical radius of curvature along a first meridian direction. The at least one second lens portion is integrally connected with a lower edge of the first lens portion, and includes a second convex surface that is distal to the wearer's face. The second convex surface has a second vertical radius of curvature along a second meridian direction. The second vertical radius of curvature is different from the first vertical radius of curvature. The third lens portion is integrally formed with and extends upwardly from an upper edge of the first lens portion, and includes a third convex surface that is distal to the wearer's face. The third convex surface has a third vertical radius of curvature along a third vertical direction. The third vertical radius of curvature is different from the first vertical radius of curvature.

Other features and advantages of the disclosure will become apparent in the following detailed description of the embodiment with reference to the accompanying drawings. It is noted that various features may not be drawn to scale.
FIG. 1 is a perspective view illustrating a goggle with an embodiment of a goggle lens according to the disclosure enclosed a wearer's head.
FIG. 2 is a perspective view of the embodiment.
FIG. 3 is a front view of the embodiment.
FIG. 4 is a sectional view taken along line IV-IV of FIG. 3.
FIG. 5 is a sectional view taken along line V-V of FIG. 3.
FIG. 6 is a sectional view taken along line VI-VI of FIG. 3.
FIG. 7 is a vertically sectional view of the embodiment.
FIG. 8 is a fragmentary sectional view illustrating the embodiment including a laminated structure.
FIG. 9 is a perspective view illustrating a lens blank from which the embodiment is cut.
FIG. 10 is a front view illustrating the lens blank from which the embodiment is cut.
FIG. 11 is a schematic side view illustrating the lens blank from which the embodiment is cut.

It should be noted herein that for clarity of description, spatially relative terms such as "top," "bottom," "upper," "lower," "on," "above," "over," "downwardly," "upwardly" and the like may be used throughout the disclosure while making reference to the features as illustrated in the drawings. The features may be oriented differently (e.g., rotated 90 degrees or at other orientations) and the spatially relative terms used herein may be interpreted accordingly.

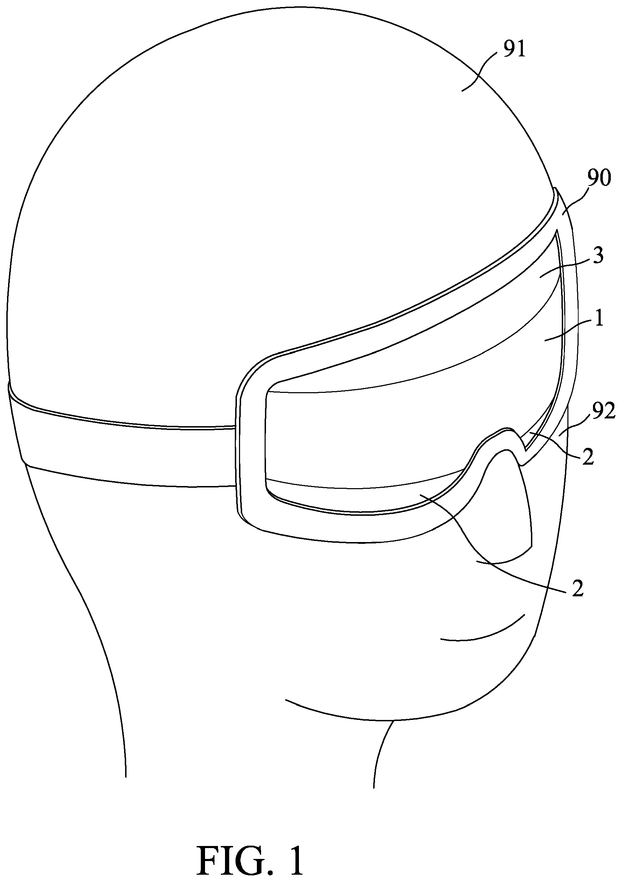

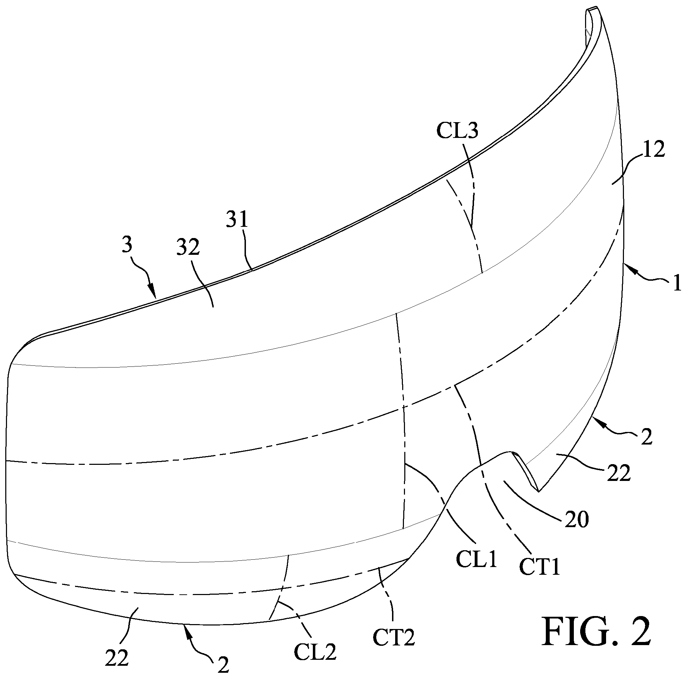

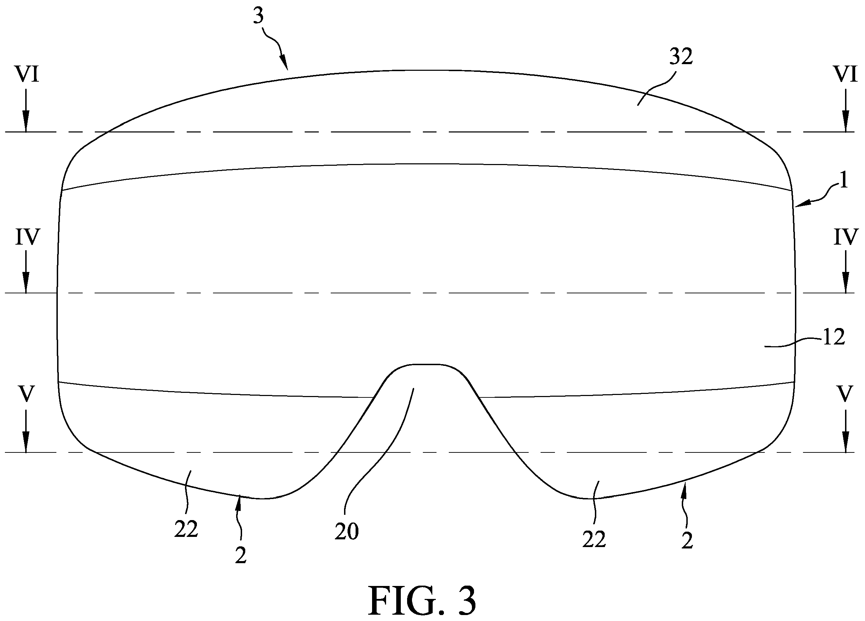

Referring to FIGS. 1 to 3, an embodiment of a goggle lens is adapted to be coupled with a goggle frame 90 for enclosing a wearer's head 91. The goggle lens has a center defined at a symmetrical center of the wearer's eyes, and two opposite sides defined at left and right sides of the wearer's eyes. The goggle lens includes a first lens portion 1, two second lens portions 2 and a third lens portion 3 integrally connected with the first lens portion 1.

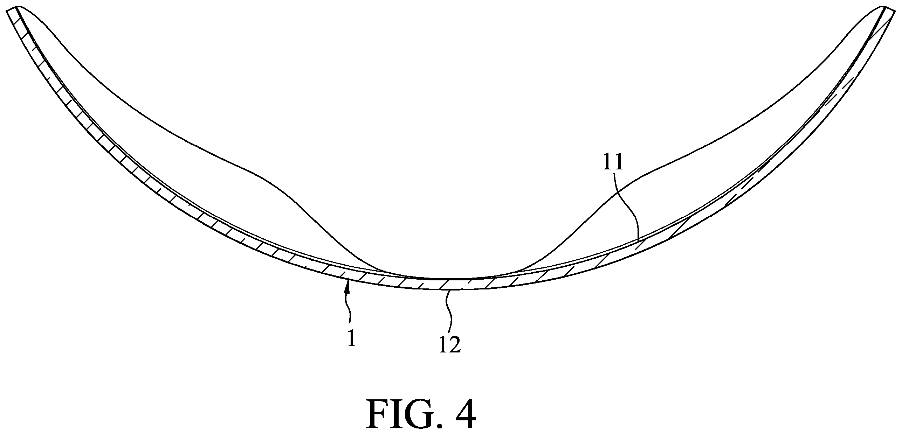

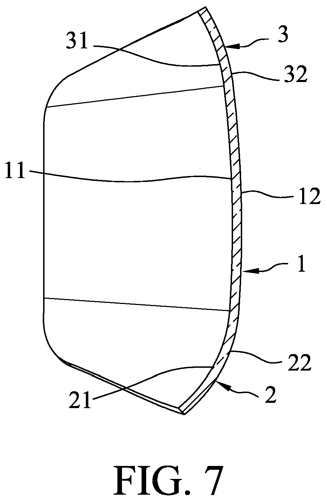

With reference to FIGS. 1, 2, 4, 7 and 11, the first lens portion 1 includes a first concave surface 11 that faces the wearer's face 92, and a first convex surface 12 that is distal to the wearer's face 92 and opposite to the first concave surface 11. The first convex surface 12 has a first vertical radius (ROC11) of curvature along a first meridian direction (as indicated by the first longitudinal curved line (CL1)), and a first horizontal radius (not shown) of curvature along a first horizontal direction (as indicated by the first transverse curved line (CT1)). The first horizontal radius of curvature is different from the first vertical radius of curvature (ROC11). The first vertical radius of curvature (ROC11) is substantially constant from the center of the lens to one of the two opposite sides of the lens.

Specifically, the first vertical radius of curvature (ROC11) is measured by: a curvature of the vertical normal section between a longitudinal normal plane (e.g. a vertical normal plane) and any point on the first convex surface 12. In the embodiment, the vertical normal section is the first longitudinal curved line (CL1). Thus, the first vertical radius of curvature (ROC11) of any point at the first longitudinal curved line (CL1) is substantially constant. The first vertical radius of curvature (ROC11) may be about 300 cm. The first horizontal radius of curvature is measured by: a curvature of the horizontal normal section between a transverse normal plane (e.g. a horizontal normal plane) and any point on the first convex surface 12. The transverse normal plane is perpendicular to the longitudinal normal plane. In the embodiment, the horizontal normal section is the first transverse curved line (CT1). Thus, the first horizontal radius of curvature of any point at the first transverse curved line (CT1) is substantially constant.

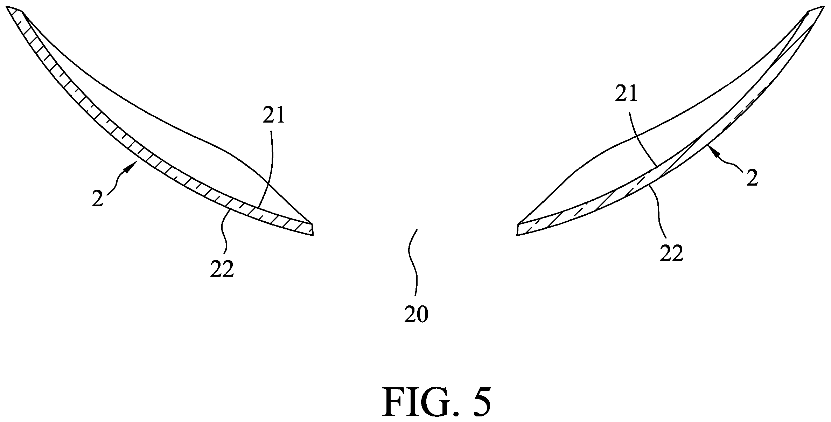

With reference to FIGS. 1, 2, 5 and 7, the two second lens portions 2 are integrally connected with and extend downwardly from a lower edge of the first lens portion 1 at left and right sides of the lower edge of the first lens portion 1, and are partially spaced apart from each other by a nose recess 20 for accommodating the wearer's nose. Each of the second lens portions 2 includes a second concave surface 21 that faces the wearer's face 92, and a second convex surface 22 that is distal to the wearer's face 92 and opposite to the second concave surface 21. With reference to FIGS. 1, 2, 5 and 11, the second convex surface 22 has a second vertical radius (ROC12) of curvature along a second meridian direction (as indicated by the second longitudinal curved line (CL2)), and a second horizontal radius (not shown) of curvature along a second horizontal direction (as indicated by the second transverse curved line (CT2)). The second horizontal radius of curvature is different from the second vertical radius of curvature (ROC12). The second vertical radius of curvature (ROC12) is different from the first vertical radius of curvature (ROC11). The second vertical radius of curvature (ROC12) of each second convex surface 22 is substantially constant from the center of the lens to the corresponding side of the lens.

Specifically, the second vertical radius of curvature (ROC12) is measured by: a curvature of the vertical normal section between a longitudinal normal plane (e.g. a vertical normal plane) and any point on the second convex surface 22. In the embodiment, the vertical normal section is the second longitudinal curved line (CL2). Thus, the second vertical radius of curvature (ROC12) of any point at the second longitudinal curved line (CL2) is substantially constant. The second vertical radius of curvature (ROC12) may be about 37.5 cm. The second horizontal radius of curvature is measured by: a curvature of the horizontal normal section between a transverse normal plane (e.g. a horizontal normal plane) and any point on the second convex surface 22. The transverse normal plane is perpendicular to the longitudinal normal plane. In the embodiment, the horizontal normal section is the second transverse curved line (CT2). Thus, the second horizontal radius of curvature of any point at the second transverse curved line (CT2) is substantially constant.

The second vertical radius of curvature (ROC12) of each second lens portion 2 may be smaller than the first vertical radius of curvature (ROC11) of the first lens portion 1. In some embodiments, a ratio of the second vertical radius of curvature (ROC12) to the first vertical radius of curvature (ROC11) is not smaller than 1/10.

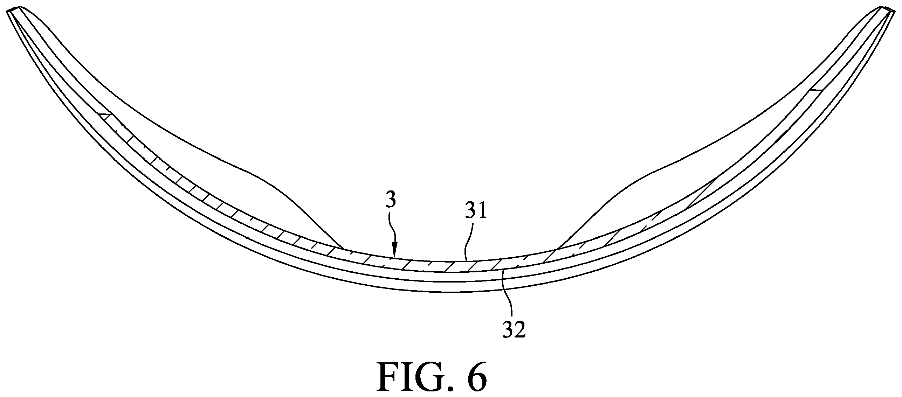

With reference to FIGS. 1, 2, 6, 7 and 11, the third lens portion 3 is integrally formed with and extends upwardly from an upper edge of the first lens portion 1, and includes a third concave surface 31 that faces the wearer's face 92, and a third convex surface 32 that is distal to the wearer's face 92 and opposite to the third concave surface 31. The third convex surface 32 has a third vertical radius (ROC13) of curvature along a third vertical direction (as indicated by the third longitudinal curved line (CL3)). The third vertical radius of curvature (ROC13) is substantially constant from the center of the lens to each of the left and right sides of the lens.

Specifically, the third vertical radius of curvature (ROC13) is measured by: a curvature of the vertical normal section between a longitudinal normal plane (e.g. a vertical normal plane) and any point on the third convex surface 32. In the embodiment, the vertical normal section is the third longitudinal curved line (CL3). Thus, the third vertical radius of curvature (ROC13) of any point at the third longitudinal curved line (CL3) is substantially constant. The third vertical radius of curvature (ROC13) may be about 50 cm.

The third vertical radius of curvature (ROC13) of the third lens portion 3 may be smaller than the first vertical radius of curvature (ROC11) of the first lens portion 1. In some embodiments, the ratio of the third vertical radius of curvature (ROC13) to the first vertical radius of curvature (ROC11) may be not smaller than 1/6.

In some embodiments, the third vertical radius of curvature (ROC13) of the third convex surface 32 gradually varies from the center of the lens to each of the left and right sides of the lens. For example, it may gradually increase, gradually decrease, gradually increase then gradually decrease, or gradually decrease then gradually increase, etc.

A smooth curved surface is formed between the first convex surface 12 and each second convex surface 22, and a smooth curved surface is formed between the first convex surface 12 and the third convex surface 32. A smooth curved surface is formed between the first concave surface 11 and each second convex surface 22, and a smooth curved surface is formed between the first concave surface 11 and the third convex surface 32.

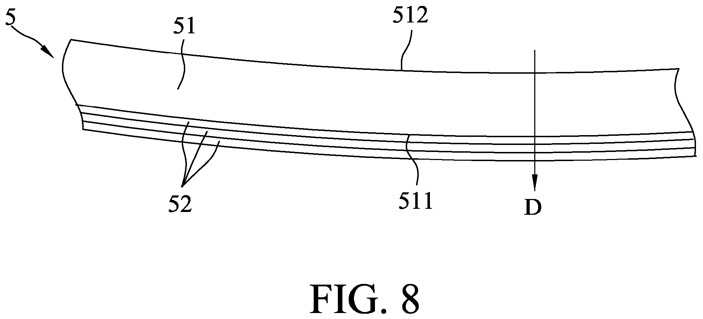

With reference to FIG. 8, moreover, the goggle lens may be made from a continuous and seamless laminated structure 5. The laminated structure 5 includes a transparent substrate 51 and a plurality of optical films 52 superimposed upon the transparent substrate 51 along a thickness direction (D) of the laminated structure 5. The transparent substrate 51 includes a front surface 511 and a back surface 512 opposite to each other. The transparent substrate 51 may be a plastic sheet made from polycarbonate (PC) and/or acrylic resin. The transparent substrate 51 may further include additives for improving the mechanical performance or the optical performance thereof.

The optical films 52 are laminated on the front surface 511 of the transparent substrate 51. In some embodiments, the optical films 52 may be laminated on the back surface 512 of the transparent substrate 51. Alternatively, in some embodiments, part of the optical films 52 are laminated on the front surface 511 of the transparent substrate 51 and the remaining part of the optical films 52 are laminated on the back surface 512 of the transparent substrate 51. The optical films 52 cooperate with the transparent substrate 51 to provide predetermined optical and physical properties. The optical films 52 may be formed by, but is not limited to, coatings, bath protective coatings or plating techniques. Said optical or physical properties may be, but are not limited to, coloration, polarization, anti-reflection, anti-fogging, scratch resistance and abrasion resistance. Since the materials used and the laminated relationship among the transparent substrate 51 and the optical films 52 are of a known type and are not the technical focus of this case, a detailed description thereof will not be provided herein.

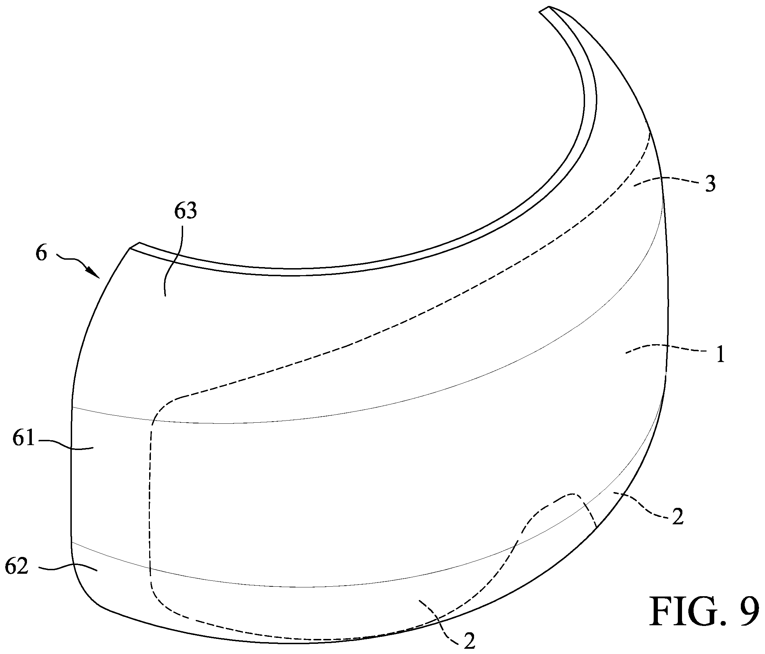

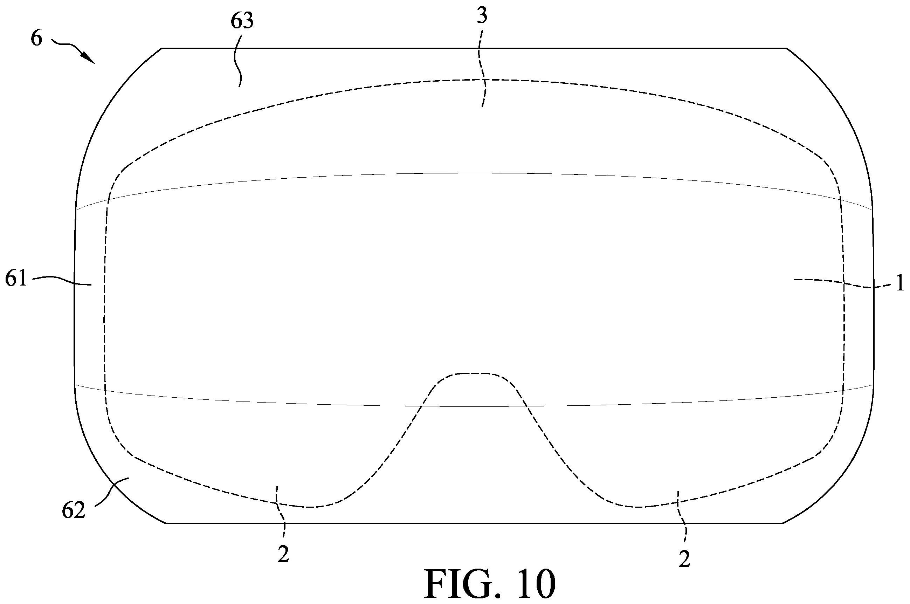

With reference to FIGS. 8 to 10, the steps of making the goggle lens are to prepare a lens blank 6, and to cut out a predetermined shape on the lens blank 6. The lens blank 6 includes a first blank portion 61, a second blank portion 62 integrally formed and connected with a lower edge of the first blank portion 61, and a third blank portion 63 integrally formed and connected with an upper edge of the first blank portion 61. The first blank portion 61 may serve as the first lens portion 1, the second blank portion 62 may serve as the second lens portions 2, and the third blank portion 63 may serve as the third lens portion 3.

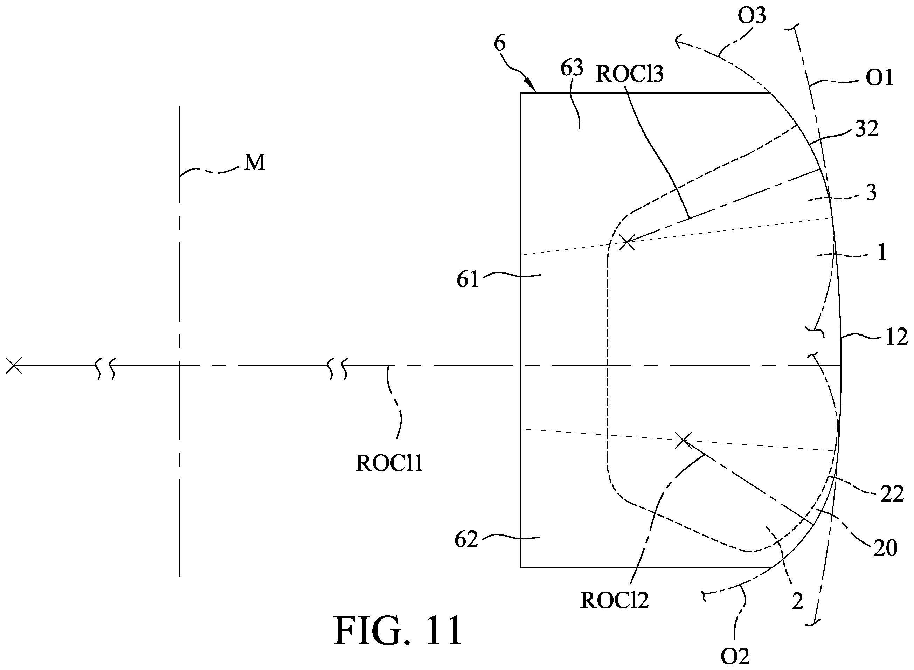

Specifically, with reference to FIG. 11, the first convex surface 12 is a part of a curved surface formed by a moving path of a first imaging circle (O1) having the first vertical radius of curvature (ROC11) around a vertical axis (M) and formed as a surface of a positive Gaussian curvature. The curved surface is a surface of a torus, and is a double curvature surface. Each second convex surface 22 is a part of a curved surface formed by a moving path of a second imaging circle (O2) having the second vertical radius of curvature (ROC12) around a vertical axis (M) and formed as a surface of a positive Gaussian curvature. The curved surface is a surface of a torus, and is a double curvature surface. The third convex surface 32 is a part of a curved surface formed by a moving path of a third imaging circle (O3) having the third vertical radius of curvature (ROC13) around a vertical axis (M) and formed as a surface of a positive Gaussian curvature. The curved surface is a surface of a torus, and is a double curvature surface. In the embodiment, the sum of the distance between the vertical axis (M) and the center of the first imaging circle (O1), the distance between the vertical axis (M) and the center of the second imaging circle (O2) and the distance between the vertical axis (M) and the center of the third imaging circle (O3) may be about 99 cm.

With reference to FIGS. 1, 2 and 11, in use, the goggle lens is mounted on the lens frame 90 to be formed as a protective goggle, such as a goggle used for athletic play or a snow sports. The thickness of the lens is decreased gradually from the center to both the left and right sides. Since the mounting of the lens and the thickness of the lens are of a known type and are not the technical focus of this case, a detailed description thereof will not be provided herein.

Through the first convex surface 12, the second convex surfaces 22 and the third convex surface 32, the goggle lens can provide the user 91 (as shown in FIG. 1) with better visual effects and a clearer and non-deformed field of vision.

Specifically, the second vertical radius of curvature (ROC12) is different from the first vertical radius of curvature (ROC11), and the third vertical radius of curvature (ROC13) is different from the first vertical radius of curvature (ROC11). The lens can provide the wearer with a better visual perception when the wearer 91 is looking up or down during skiing on snow. Moreover, with the second vertical radius of curvature (ROC12) of each second convex surface 22 being constant from the center to the corresponding side and with the second lens portions 2 having a constant thickness, image distortion which may cause dizziness to the wearer 91 is prevented when the wearer 91 is looking down through the lens. This improves the safety of the goggle and the eye comfort of the wearer 91.

Additionally, it should be noted that the number of the second lens portions 2 may be varied pertaining to different requirements. For example, only one second lens portion 2 may be provided and formed on the lower edge of the first lens portion 1.

## Claims

1. A goggle lens adapted to be coupled with a goggle frame (90) for enclosing a wearer's head (91), comprising:
a first lens portion (1) including a first convex surface (12) that is distal to the wearer's face, said first convex surface (12) having a first vertical radius of curvature (ROC11) along a first meridian direction;
at least one second lens portion (2) integrally connected with a lower edge of said first lens portion (1), said at least one second lens portion (2) including a second convex surface (22) that is distal to the wearer's face, said second convex surface (22) having a second vertical radius of curvature (ROC12) along a second meridian direction; and
a third lens portion (3) integrally formed with and extending upwardly from an upper edge of said first lens portion (1), said third lens portion (3) including a third convex surface (32) that is distal to the wearer's face, said third convex surface (32) having a third vertical radius of curvature (ROC13) along a third vertical direction, **characterized in that**:
said second vertical radius of curvature (ROC12) is different from said first vertical radius of curvature (ROC11), and said third vertical radius of curvature (ROC13) is different from said first vertical radius of curvature (ROC11).

2. The goggle lens of claim 1, wherein said goggle lens has a center defined at a symmetrical center of the wearer's eyes, and two opposite sides defined at left and right sides of the wearer's eyes, wherein said first vertical radius of curvature (ROC11) is substantially constant from said center to one of said two opposite sides,
said first convex surface (12) further having a first horizontal radius of curvature along a first horizontal direction, wherein said first horizontal radius of curvature is different from said first vertical radius of curvature (ROC11).

3. The goggle lens of claim 1, wherein said at least one second lens portion (2) is two second lens portions (2), each of which integrally extends downwardly from said lower edge of said first lens portion (1), said two second lens portions (2) being partially spaced apart from each other by a nose recess (20).

4. The goggle lens of claim 3, wherein said second convex surface (22) of each of said two second lens portions (2) further has a second horizontal radius of curvature along a second horizontal direction, wherein said second horizontal radius of curvature is different from said second vertical radius of curvature (ROC12).

5. The goggle lens of claim 3, wherein said goggle lens has a center defined at a symmetrical center of the wearer's eyes, and two opposite sides defined at left and right sides of the wearer's eyes, and said second vertical radius of curvature (ROC12) of each of said second convex surfaces (22) is substantially constant from said center to one of said two opposite sides.

6. The goggle lens of claim 1, wherein said goggle lens has a center defined at a symmetrical center of the wearer's eyes, and two opposite sides defined at left and right sides of the wearer's eyes, and said third vertical radius of curvature (ROC13) of said third convex surface (32) is substantially constant from said center to one of said two opposite sides.

7. The goggle lens of claim 1, wherein said goggle lens has a center defined at a symmetrical center of the wearer's eyes, and two opposite sides defined at left and rig ht sides of the wearer's eyes, and said third vertical radius of curvature (ROC13) of said third convex surface (32) gradually varies from said center to one of said two opposite sides.

8. The goggle lens of claim 1, wherein said third vertical radius of curvature (ROC13) of said third convex surface (32) is smaller than said first vertical radius of curvature (ROC11) of said first convex surface (12), and a ratio of said third vertical radius of curvature (ROC13) to said first vertical radius of curvature (ROC11) is not smaller than 1/6.

9. The goggle lens of claim 1, wherein said second vertical radius of curvature (ROC12) of said second convex surface (22) is smaller than said first vertical radius of curvature (ROC11) of said first convex surface (12), and a ratio of said second vertical radius of curvature (ROC12) to said first vertical radius of curvature (ROC11) is not smaller than 1/10.
